# EUROPEAN PATENT APPLICATION

(11) **EP 4 718 378 A1**
(43) Date of publication of application: **01.04.2026**
(21) Application number: 25205063.8
(22) Date of filing: 26.09.2025
(51) Int. Cl.: G06T 7/00, G06T 7/20

(54) **MEDICAL INFORMATION PROCESSING APPARATUS AND MEDICAL INFORMATION PROCESSING METHOD**

(30) Priority: 30.09.2024 JP 2024170825
(71) Applicant: Canon Medical Systems Corporation, Tochigi 324-0036 (JP)
(72) Inventor: KAWASHIMA, Keitaro, Otawara-shi 324-0036 (JP); AOYAMA, Gakuto, Otawara-shi 324-0036 (JP)
(74) Representative: Marks & Clerk LLP

(57) **Abstract**

A medical information processing apparatus (100) according to an embodiment includes processing circuitry (150). The processing circuitry (150) acquires a plurality of images of a target organ (20) captured in time series. The processing circuitry (150) specifies, from each of the images, a target organ region (200) where the target organ (20) is depicted. The processing circuitry (150) specifies, from each of the images, a device region (60) where a device (30) attached to the target organ (20) is depicted. The processing circuitry (150) specifies, based on the target organ region (200) and the device region (60), an index relating to a state of the target organ (20) to which the device (30) is attached. The index is specified from change in a relative position of the device (30) with respect to the target organ (20) in a specific attention direction (D). An embodiment discloses a medical information processing method.

## Description

### FIELD

Embodiments described herein relate generally to a medical information processing apparatus and a medical information processing method.

### BACKGROUND

Conventionally, techniques for attaching a device for treatment to an organ of a patient have been known. For example, as an treatment for secondary mitral regurgitation (MR) of the heart, a technique of attaching a clip-shaped device serving to grip the tip ends of the anterior leaflet and the posterior leaflet of the mitral valve to reduce regurgitation of blood flow has been known.

In such a technique, the device applies a load to the mitral valve and restricts the movement of the mitral valve, and the load changes with the attachment position of the device. While performing a procedure, a physician determines the attachment position of the device after deciding positions where the load can be kept within an appropriate range. Conventionally, for example, the physician estimates the load on the mitral valve by observing transesophageal echocardiography (TEE) images and visually and qualitatively evaluating the magnitude of pressure on the mitral valve or the magnitude of tension of the mitral valve.

### SUMMARY OF THE INVENTION

A medical information processing apparatus is provided, which includes processing circuitry. The processing circuitry may be configured to: acquire a plurality of images of a target organ captured in time series; specify, in each of the images, a target organ region where the target organ is depicted; specify, in each of the images, a device region where a device attached to the target organ is depicted; and specify, based on the target organ region and the device region, an index relating to a state of the target organ to which the device is attached, the index being specified from change in a relative position of the device with respect to the target organ in a specific attention direction.

The images may be three-dimensional images of the target organ captured in time series. The processing circuity may be further configured to specify, in each of the images, attention coordinates being three-dimensional coordinates of one point included in the device region, and specify, in each of the images, a long axis direction of the device in a three-dimensional space as the attention direction based on the device region. The index may be an amount of variation of a distance between the attention coordinates and the target organ region in the attention direction.

The processing circuitry may be further configured to acquire, at different time points, the images of the target organ captured in time series, specify average coordinates of the target organ at each of the different time points, the average coordinates being specified from the target organ region specified in each of the images, specify a maximum position and a minimum position in the attention direction from among the average coordinates at each of the different time points, and calculate, as the amount of variation, an absolute value of a difference between a first distance and a second distance, the first distance being a distance between the attention coordinates and the maximum position in the attention direction, the second distance being a distance between the attention coordinates and the minimum position in the attention direction.

The processing circuitry may be configured to specify, as the attention direction, a first principal component obtained through a principal component analysis with respect to coordinate information about each pixel included in the device region specified in each of the images.

The processing circuitry may be further configured to specify the device region by three feature points corresponding to three edge points of the device, specify, as the attention coordinates, a center of gravity of the three feature points, and specify, as the attention direction, an extending direction of a line segment from the attention coordinates toward a midpoint, the line segment connecting between the attention coordinates and the midpoint that connects respective three-dimensional coordinates of two feature points corresponding to two edge points in a short-length direction of the device among the three feature points.

The processing circuitry may be further configured to display, on a display, the index along with an image indicating the maximum position and the minimum position of the attention coordinates, or display, on the display, a graph indicating a time-series change in the calculated index.

The processing circuitry may be further configured to correct the index in accordance with the size of the device or the number of the devices.

The processing circuitry may be further configured to specify a reference plane of the target organ, specify, in each of the images, attention coordinates being three-dimensional coordinates of one point included in the device region, and specify a normal direction of the reference plane as the attention direction. The index relating to the state of the target organ may be an amount of variation of a distance between the reference plane and the attention coordinates in the attention direction.

The target organ may be a mitral valve of the heart. The device may serve to grip tip ends of an anterior leaflet and a posterior leaflet of the mitral valve. The index may be a numerical value indirectly indicating tension of the mitral valve in a state of being gripped by the device.

A medical information processing method implemented by a computer is provided. The method may include: acquiring a plurality of images of a target organ captured in time series; specifying, in each of the images, a target organ region where the target organ is depicted; specifying, in each of the images, a device region where a device attached to the target organ is depicted; and specifying, based on the target organ region and the device region, an index relating to a state of the target organ to which the device is attached, the index being specified from change in a relative position of the device with respect to the target organ in a specific attention direction.

### BRIEF DESCRIPTION OF THE DRAWINGS

FIG. 1 is a diagram illustrating an example of a state where a procedure of attaching a device to a mitral valve of the heart is being performed;
FIG. 2 is a diagram illustrating an example of a state where the device is attached to the mitral valve;
FIG. 3 is a diagram illustrating an example of a configuration of a medical image processing apparatus according to an embodiment;
FIG. 4 is a flowchart illustrating an example of a procedure of calculation processing of an index relating to the state of the mitral valve according to the embodiment;
FIG. 5 is a diagram illustrating an example of a four-dimensional image according to the embodiment;
FIG. 6 is a diagram illustrating another example of the four-dimensional image according to the embodiment;
FIG. 7 is a diagram illustrating an example of a method for specifying a mitral valve region as a mesh according to the embodiment;
FIG. 8 is a diagram illustrating an example of the definition of a mesh indicating the mitral valve region according to the embodiment;
FIG. 9 is a diagram illustrating an example of time-series changes in the shape of a mitral valve mesh representing the mitral valve region according to the embodiment;
FIG. 10 is a diagram illustrating an example of a method for specifying the mitral valve region as a region according to the embodiment;
FIG. 11 is a diagram illustrating an example of a device region 60 specified as a domain according to the embodiment;
FIG. 12 is a diagram illustrating an example of a method for specifying a device region as a mesh according to the embodiment;
FIG. 13 is a diagram illustrating an example of a method for specifying the device region as a region according to the embodiment;
FIG. 14 is a diagram illustrating an example of the device region 60 specified by respective three-dimensional coordinates of plural points according to the embodiment;
FIG. 15 is a diagram illustrating an example of feature points of a device according to the embodiment;
FIG. 16 is a diagram illustrating an example of feature points when the device is closed more firmly than that in FIG. 15;
FIG. 17 is a diagram illustrating an example of feature points when the device is opened more widely than that in FIG. 15;
FIG. 18 is a diagram illustrating an example of time-series changes in the positions of the three-dimensional coordinates that define the device region according to the embodiment;
FIG. 19 is a diagram illustrating an example of attention coordinates according to the embodiment;
FIG. 20 is a diagram illustrating an example of time-series changes in the position of the attention coordinates according to the embodiment;
FIG. 21 is a diagram illustrating an example of an attention direction according to the embodiment;
FIG. 22 is a diagram illustrating an example of a specification method of an attention direction D, when the device region is specified as a domain according to the embodiment;
FIG. 23 is a diagram illustrating an example of time-series changes in the attention direction according to the embodiment;
FIG. 24 is a diagram schematically illustrating an example of coordinates of each pixel included in the mitral valve region in a three-dimensional image according to the embodiment;
FIG. 25 is a diagram illustrating an example of the average coordinate position of coordinates of each pixel included in an anterior leaflet region of the mitral valve region in the three-dimensional image according to the embodiment;
FIG. 26 is a diagram illustrating an example of positions of the average coordinate positions at times with respect to the attention direction according to the embodiment;
FIG. 27 is a diagram illustrating an example of a display mode for an amount of variation according to the embodiment; and
FIG. 28 is a diagram illustrating an example of a specification method of three-dimensional distance between the mitral valve and the device between time phases according to a first modification.

### DETAILED DESCRIPTION

Hereinafter, embodiments of a medical information processing apparatus and a medical information processing method will be described in detail with reference to the accompanying drawings. However, the medical information processing apparatus and the medical information processing method according to the present application are not limited to the embodiments described below. Although plural features are described in the embodiments, not all the features are essential. The features may be optionally combined. In the accompanying drawings, the same reference numerals denote the same or similar components, and repeated descriptions thereof will be omitted.

A medical information processing apparatus according to an embodiment includes processing circuitry. The processing circuitry is configured to acquire a plurality of images of a target organ captured in time series. The processing circuitry is configured to specify, in each of the images, a target organ region where the target organ is depicted. The processing circuitry is configured to specify, in each of the images, a device region where a device attached to the target organ is depicted. The processing circuitry is configured to specify, based on the target organ region and the device region, an index relating to a state of the target organ to which the device is attached. The index is specified from change in a relative position of the device with respect to the target organ in a specific attention direction.

The medical information processing apparatus and the medical information processing method according to the present embodiment are used for calculating an index that indirectly indicates the load on the mitral valve leaflet at a location where a device for treatment is attached. Here, a treatment method of mitral valve repair by attaching a device will be described.

Various treatment methods have been developed to treat cardiac disease. For example, for secondary mitral regurgitation (MR), a physician uses a catheter to treat a patient who is a subject by attaching a device to the mitral valve with a transvascular approach.

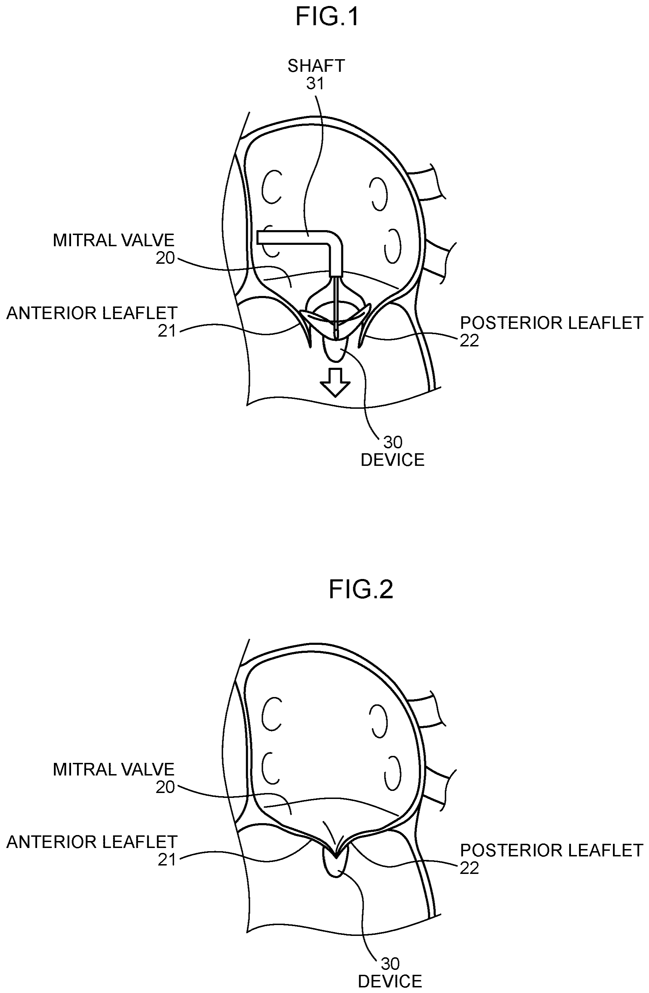

FIG. 1 is a diagram illustrating an example of a state where a procedure of attaching a device 30 to a mitral valve 20 of the heart is being performed. By operating a shaft 31, a physician attaches the device 30 to the mitral valve 20. The device 30 is a type of a mitral valve repair device, and can increase the joining region by gripping the tip ends of an anterior leaflet 21 and a posterior leaflet 22 of the mitral valve 20.

Such a method is referred to as an edge-to-edge repair. The device 30 used for edge-to-edge repair includes MitraClip (registered trademark), PASCAL (Edwards Lifesciences), etc.

FIG. 2 is a diagram illustrating an example of a state where the device 30 is attached to the mitral valve 20. When the physician attaches the device 30 to an appropriate position, and then releases the shaft 31, the device 30 is attached to the mitral valve 20 in a state in which the tip ends of the anterior leaflet 21 and the posterior leaflet 22 of the mitral valve 20 are being gripped. The attachment position of the device 30 can be changed until the shaft 31 is released. However, once the shaft 31 is released, the device 30 is fixed at the position where the shaft 31 is released. Therefore, in general, the physician releases the shaft 31, after carefully checking the attachment position of the device 30 through transesophageal echocardiography (TEE) images and the like where the mitral valve 20 is depicted.

Transcatheter mitral valve repair with the device 30 is an approved treatment method widely used for such secondary mitral regurgitation patients, and the safety and efficacy is supported by large-scale randomized clinical trials. However, despite the track record of safety, a small, albeit important number of mitral valve leaflet adverse events (LAE) regarding the treatment device have been reported. The types of LAE include leaflet perforation, leaflet tear, leaflet shape distortion, partial leaflet gripping, chordal entrapment, and the like. As an example of a possible cause of LAE such as leaflet tear, it is considered that the load such as tension on the mitral valve 20 is increased by attaching the device 30 that restrains the periodic movement of the mitral valve 20 that moves periodically.

Also, there is a technique of calculating in advance, by computer simulation, the appropriate attachment position of the device 30 or the load such as blood flow on the mitral valve 20. However, it may be difficult to predict how far the mitral valve 20 can withstand the load with high accuracy in advance. This is because the durability differs with the state of the individual patient. Moreover, for determining whether to release the shaft 31, the physician is required to check the state of the mitral valve 20 at real time during the procedure. The medical information processing apparatus and the medical information processing method of the present embodiment can be used for providing an index relating to the state of the mitral valve 20 to a physician in the above-described cases.

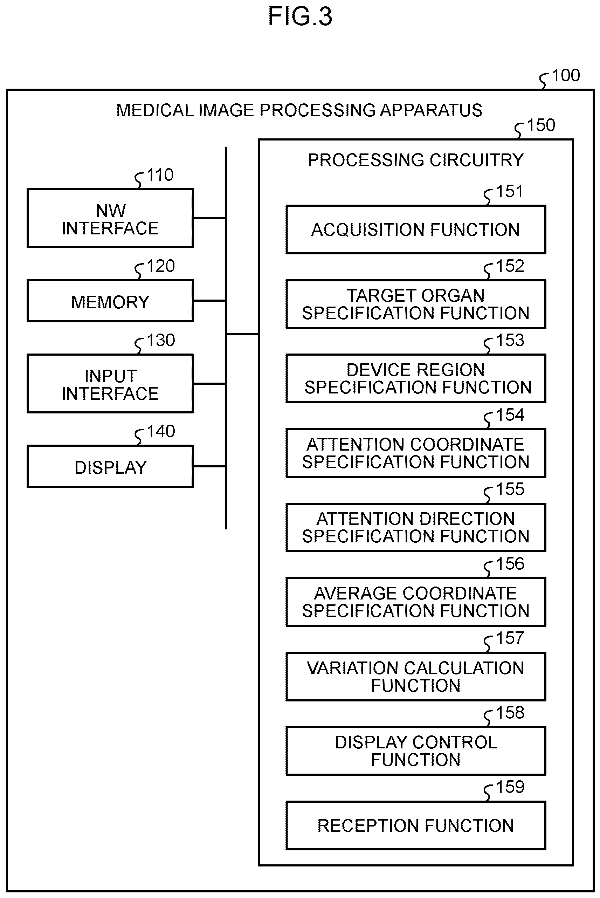

FIG. 3 is a diagram illustrating an example of a configuration of a medical image processing apparatus 100 (an example of the medical information processing apparatus) according to the embodiment. The medical image processing apparatus 100 may be a computer such as a server or a personal computer (PC).

As illustrated in FIG. 3, the medical image processing apparatus 100 includes a network (NW) interface 110, a memory 120, an input interface 130, a display 140, and processing circuitry 150.

The NW interface 110 is connected to the processing circuitry 150. The NW interface 110 controls transmission and communication of various data between the medical image processing apparatus 100 and other devices. The other devices may include, for example, medical image storage devices such as a picture archiving and communication system (PACS) that stores medical image data, various types of modalities (medical imaging apparatus), electronic chart systems, and the like. The NW interface 110 is implemented by a network card, a network adapter, a network interface controller (NIC), and the like.

The memory 120 stores in advance various types of information used by the processing circuitry 150. The memory 120 stores various computer programs. The memory 120 may be a non-volatile storage device such as a hard disk drive (HDD), a solid-state drive (SSD), or an integrated circuit storage device that stores various types of information. In addition to the HDD, the SSD, etc., the memory 120 may be a portable storage medium such as a compact disc (CD), a digital versatile disc (DVD), and a flash memory, or a driving device that reads and writes various types of information to and from a semiconductor memory element such as a random access memory (RAM). The memory 120 is an example of a storage unit.

The input interface 130 is implemented by using a mouse, a keyboard, a pen tablet that is a combination of a touch pen and a tablet that receive user operations, a trackball, a switch button, a touch pad with which input operations are performed by touching an operation surface, a touch screen in which a display screen and a touch pad are integrated, a non-contact input circuit using an optical sensor, a voice input circuit, and the like. The input interface 130 may include a plurality of devices that receive user operations. The input interface 130 is connected to the processing circuitry 150, converts an input operation received from the user into an electrical signal, and outputs the converted electrical signal to the processing circuitry 150. In the present specification, the input interface is not limited to one having physical operation parts such as a mouse and a keyboard. For example, processing circuitry of electrical signals that receives an electrical signal corresponding to an input operation from an external input device, which is provided separately from the apparatus, and that outputs the electrical signal to the processing circuitry 150, is also included in an example of the input interface.

Under control of the processing circuitry 150, the display 140 displays various types of information. For example, the display 140 outputs various generated images, a graphical user interface (GUI) for receiving various operations from the user, and the like. Specifically, the display 140 is a liquid crystal display, a cathode ray tube (CRT) display, and the like. The input interface 130 and the display 140 may be integrated with each other. For example, the input interface 130 and the display 140 may be implemented by a touch panel.

The processing circuitry 150 is a processor that implements a function corresponding to each computer program by reading a computer program from the memory 120 and executing the read computer program. The processing circuitry 150 of the present embodiment includes an acquisition function 151, a target organ region specification function 152, a device region specification function 153, an attention coordinate specification function 154, an attention direction specification function 155, an average coordinate specification function 156, a variation calculation function 157, a display control function 158, and a reception function 159. The acquisition function 151 is an example of an acquisition unit. The target organ region specification function 152 is an example of a target organ region specification unit. The device region specification function 153 is an example of a device region specification unit. The attention coordinate specification function 154 is an example of an attention coordinate specification unit. The attention direction specification function 155 is an example of an attention direction specification unit. The average coordinate specification function 156 is an example of an average coordinate specification unit. The variation calculation function 157 is an example of a variation calculation unit and an index specification unit. The display control function 158 is an example of a display control unit. The reception function 159 is an example of a reception unit.

In the present embodiment, each of the processing functions of the acquisition function 151, the target organ region specification function 152, the device region specification function 153, the attention coordinate specification function 154, the attention direction specification function 155, the average coordinate specification function 156, the variation calculation function 157, the display control function 158, and the reception function 159, which are the functional components of the processing circuitry 150, are stored in the memory 120 in the form of computer-executable programs, for example. The processing circuitry 150 is a processor. For example, the processing circuitry 150 implements the function corresponding to each computer program, by reading a computer program from the memory 120 and executing the read computer program. In other words, the processing circuitry 150 that has read out each computer program has each of the functions illustrated in the processing circuitry 150 in FIG. 3. In FIG. 3, the processing functions performed by the acquisition function 151, the target organ region specification function 152, the device region specification function 153, the attention coordinate specification function 154, the attention direction specification function 155, the average coordinate specification function 156, the variation calculation function 157, the display control function 158, and the reception function 159 are performed by a single processor. However, the processing circuitry 150 may be configured by combining a plurality of independent processors, and each of the processors may execute a computer program to implement the function. Moreover, in FIG. 3, a single memory 120 stores a computer program corresponding to each processing function. However, a plurality of memories may be disposed in a dispersed manner, and the processing circuitry 150 may read a corresponding computer program from individual memory.

In the above explanation, a "processor" reads a computer program corresponding to each function from the memory and executes the read computer program. However, the embodiment is not limited thereto. The term "processor" may refer to circuitry such as a central processing unit (CPU), a graphics processing unit (GPU), an application specific integrated circuit (ASIC), a programmable logic device (for example, a simple programmable logic device (SPLD) or a complex programmable logic device (CPLD)), and a field programmable gate array (FPGA). In a case where the processor is a CPU, the processor implements a function by reading and executing the computer program stored in the memory. In a case where the processor is an ASIC, instead of storing the computer program in the memory 120, the above-noted functions are incorporated directly into a circuit of the processor as a logic circuit. Each processor in the present embodiment is not limited to being configured as a single circuit for each processor, but may also be configured as a single processor by combining multiple independent circuits to implement the functions. Moreover, a plurality of components in FIG. 3 may be integrated into a single processor to implement the functions.

The acquisition function 151 acquires a plurality of images of a target organ captured in time series at different time points, from a medical image storage device or various types of modalities via the NW interface 110. The target organ is an object to which the device 30 is to be attached, and is an object whose state is to be measured while the device 30 is being attached. The target organ is assumed to be an organ that moves periodically or regularly. The device 30 has a function to restrain the periodic or regular movement of the target organ. In the present embodiment, the mitral valve 20 is an example of the target organ. The mitral valve 20 performs regular and periodic reciprocating motion. In the present embodiment, as described above, the mitral valve repair device such as MitraClip (registered trademark) or PASCAL (Edwards Lifesciences) is an example of the device 30.

The images are three-dimensional images of the mitral valve 20 captured at different time points. The images have spatial three-dimensional information and temporal dimensional (one-dimensional) information, so that such images are also referred to as a four-dimensional image. More precisely, the four-dimensional image is an image including morphological information about the three-dimensional anatomical structure of the mitral valve 20 captured continuously in time series. However, the mode of the four-dimensional image is not limited thereto, and any image including at least two or more three-dimensional images captured at different time points can be employed. In the present embodiment, the four-dimensional image of the mitral valve 20 is a 4D TEE image. The type of the three-dimensional images captured at different time points is not limited to the TEE image. The type of the three-dimensional images may also be medical images captured by various types of modalities such as a computed tomography (CT) image, a magnetic resonance imaging (MRI) image, an X-ray image, a positron emission computed tomography (PET) image, a single photon emission computed tomography (SPECT) image, etc.

The target organ region specification function 152 specifies, in the four-dimensional image acquired by the acquisition function 151, the region where the mitral valve 20 as a target organ is depicted. Specifying the region where the mitral valve 20 is depicted means to specify the three-dimensional coordinates of each pixel in the region where the mitral valve 20 is depicted. More precisely, in each of the three-dimensional images captured at different time points included in the four-dimensional image, the target organ region specification function 152 specifies the region where the mitral valve 20 is depicted. Hereinafter, the region where the mitral valve 20 is depicted is referred to as a mitral valve region. The mitral valve region is an example of a target organ region in the present embodiment.

The device region specification function 153 specifies, in the four-dimensional image acquired by the acquisition function 151, the region where the device 30 attached to the target organ is depicted. Specifying the region where the device 30 is depicted, means to specify the three-dimensional coordinates of each pixel in the region where the device 30 is depicted. More precisely, in each of the three-dimensional images captured at different time points included in the four-dimensional image, the device region specification function 153 specifies the region where the device 30 is depicted. Hereafter, the region where the device 30 is depicted is referred to as a device region.

The attention coordinate specification function 154 specifies the attention coordinates of the device 30 in the device region specified by the device region specification function 153 from each of the three-dimensional images captured at different time points included in the four-dimensional image. The attention coordinates are coordinates indicating the position of the device 30 in the three-dimensional image. The attention coordinates are three-dimensional coordinates of one point in the device region. The attention coordinates may be the center of gravity of feature points corresponding to the edges of the device 30.

In each of the three-dimensional images captured at different time points included in the four-dimensional image, the attention direction specification function 155 specifies the attention direction on the basis of the device region specified by the device region specification function 153. The attention direction refers to a direction toward which the device 30 mainly moves with the movement of the mitral valve 20. In general, the device 30 attached to the mitral valve 20 performs periodic reciprocating motion with the mitral valve 20 in the long axis direction of the device 30. Therefore, the attention direction specification function 155 specifies, as the attention direction, the long axis direction of the device 30 in the three-dimensional space.

In each of the three-dimensional images captured at different time points included in the four-dimensional image, the average coordinate specification function 156 specifies the average coordinate position of the target organ at each of the different time points. The different time points refer to points of time when the three-dimensional images included in the four-dimensional image acquired by the acquisition function 151 are respectively captured.

On the basis of the specified target organ region and the device region, the variation calculation function 157 specifies an index relating to the state of the target organ to which the device 30 is attached, on the basis of change in the relative position of the device 30 with respect to the target organ in a specific attention direction between different time points.

In the present embodiment, the amount of variation of the distance between the attention coordinates and the mitral valve region at different time points is an example of the index relating to the state of the target organ. The calculation of the amount of variation is an example of processing of specifying an index. The amount of variation of the distance between the attention coordinates and the mitral valve region at different time points differs with the magnitude of pressure or tension on the mitral valve 20. For example, when large tension is applied to the mitral valve 20 and the mitral valve 20 is stretching tightly, the movement of the mitral valve 20 is limited strongly. Hence, the amount of variation of the distance between the attention coordinates and the mitral valve region is reduced. That is, the amount of variation of the distance between the attention coordinates and the mitral valve region is reduced with an increase in the tension, and the amount of variation of the distance between the attention coordinates and the mitral valve region is increased with a reduction in the tension. Therefore, the amount of variation of the distance between the attention coordinates and the mitral valve region at different time points becomes an index that indirectly indicates the tension or the like on the mitral valve 20. Moreover, the amount of variation of the distance between the attention coordinates and the mitral valve region at different time points differs with the degree of flexibility of the mitral valve 20.

The display control function 158 causes the display 140 to display various images or GUIs. For example, by causing the display 140 to display an index relating to the state of the target organ specified by the variation calculation function 157, the display control function 158 presents the index to the physician during the procedure. The display control function 158 may cause the display 140 to display the amount of variation of the distance between the attention coordinates and the mitral valve region, with an image where the mitral valve 20 and the device 30 are depicted. Moreover, the display control function 158 may cause the display 140 to display a graph indicating time-series changes in the amount of variation of the distance between the attention coordinates and the mitral valve region.

The reception function 159 receives various user operations via the input interface 130. The reception function 159 may receive a user operation to specify the position of the mitral valve 20 in the three-dimensional image. The reception function 159 may receive a user operation to specify the position of the device 30 in the three-dimensional image. Moreover, the reception function 159 may receive a user operation to specify the attention direction in the three-dimensional image.

Calculation processing of an index relating to the state of the mitral valve 20 executed by the medical image processing apparatus 100 configured as described above will be described with reference to a flowchart.

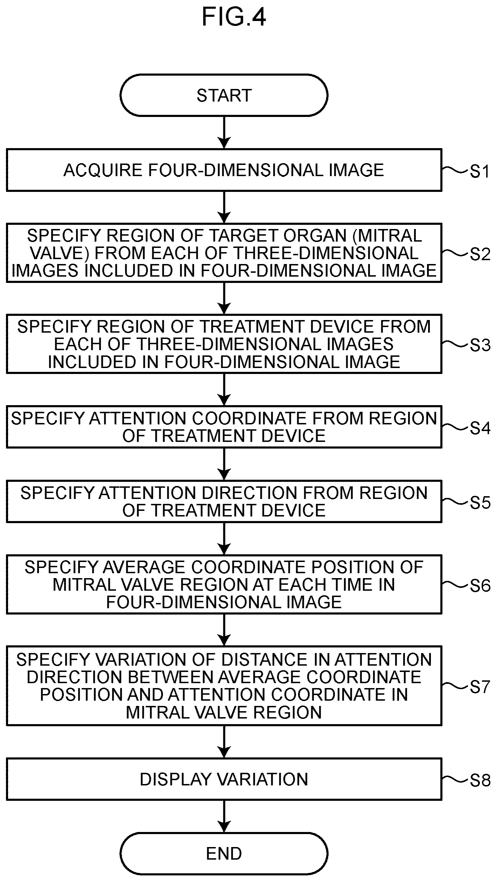

FIG. 4 is a flowchart illustrating an example of a procedure of calculation processing of an index relating to the state of the mitral valve 20 according to the embodiment. The processing of the flowchart is performed after the device 30 is attached to the mitral valve 20 but before the shaft 31 is released. In the following description, a plurality of drawings are used for explanation with the flowchart. However, the illustration of the shaft 31 is omitted in each drawing.

First, the acquisition function 151 acquires a four-dimensional image such as a 4D TEE image in which the mitral valve 20 is captured (step S1).

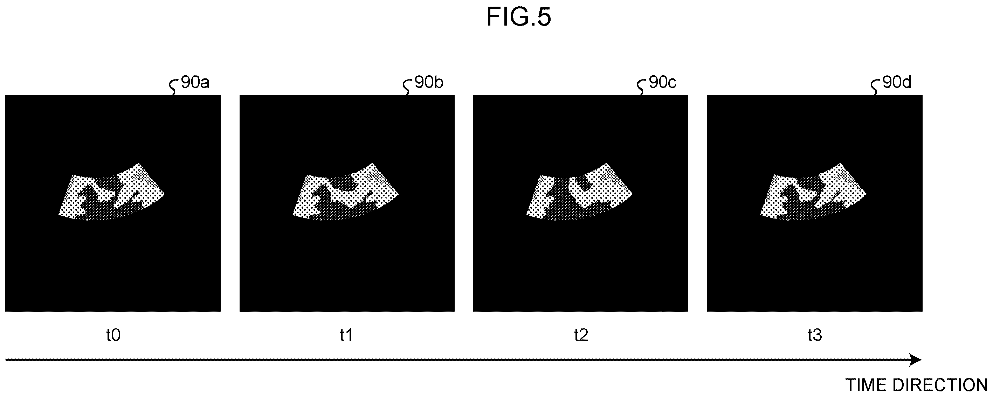

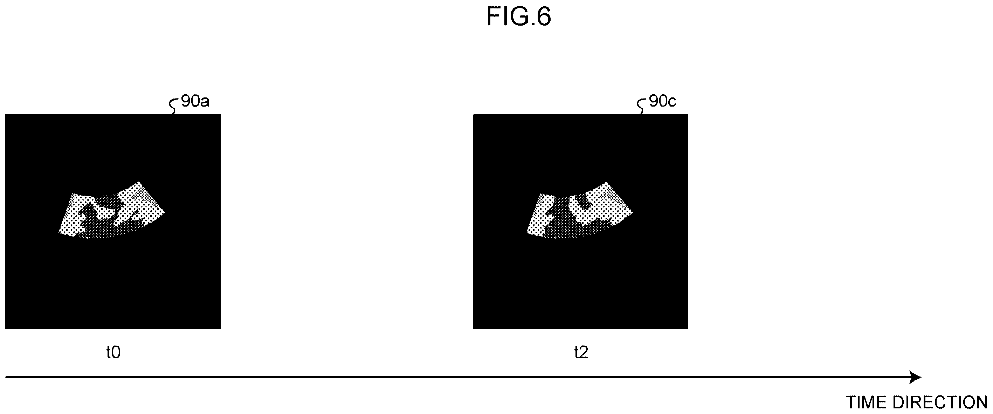

FIG. 5 is a diagram illustrating an example of a four-dimensional image according to the embodiment. As illustrated in FIG. 5, the four-dimensional image may be constituted by three-dimensional images 90a to 90d in which the mitral valve 20 is captured at consecutive times t0, t1, t2, and t3. FIG. 6 is a diagram illustrating another example of the four-dimensional image according to the embodiment. The imaging times of the three-dimensional images included in the four-dimensional image need not be continuous. For example, as illustrated in FIG. 6, the three-dimensional images 90a and 90c captured at time t0 and time t2 at intervals may constitute the four-dimensional image. In the present embodiment, as illustrated in FIG. 5, the acquisition function 151 acquires the three-dimensional images 90a to 90d in which the mitral valve 20 is captured at consecutive times t0, t1, t2, and t3.

Returning to FIG. 4, the target organ region specification function 152 specifies the region (mitral valve region) of the target organ (mitral valve 20) in each of the three-dimensional images included in the acquired four-dimensional image (step S2). For example, the target organ region specification function 152 acquires coordinate information about each pixel where the mitral valve 20 is depicted, from each of the three-dimensional images 90a to 90d included in the 4D TEE image. In other words, the coordinate information about each pixel of the mitral valve 20 is the coordinate information about each pixel included in the mitral valve region. The target organ region specification function 152 may also acquire the coordinate information about each pixel included in the mitral valve region, as a mesh or as a region (segment).

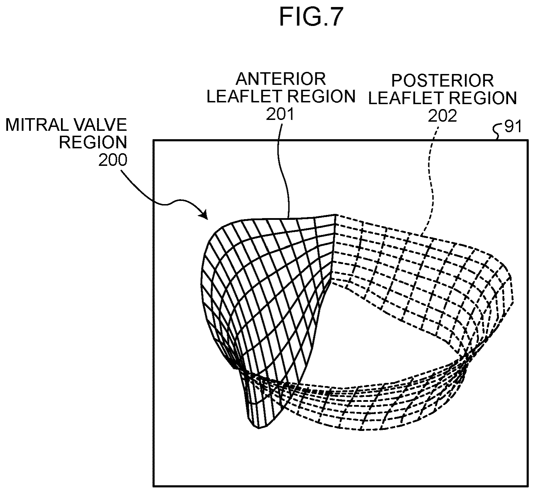

FIG. 7 is a diagram illustrating an example of a method for specifying a mitral valve region 200 as a mesh according to the embodiment. As a mesh image 91 illustrated in FIG. 7, the mitral valve region 200 can be represented by a three-dimensional mesh model. The mitral valve region 200 includes an anterior leaflet region 201 where the anterior leaflet 21 is depicted, and a posterior leaflet region 202 where the posterior leaflet 22 is depicted.

The three-dimensional mesh model is represented by a computational grid (mesh) of the mitral valve region 200 by setting a plurality of grid points on the specified mitral valve region. In this case, the number and arrangement of the grid points may be determined in advance, or may be determined by the target organ region specification function 152 on the basis of the size, shape, and the like of the mitral valve region 200.

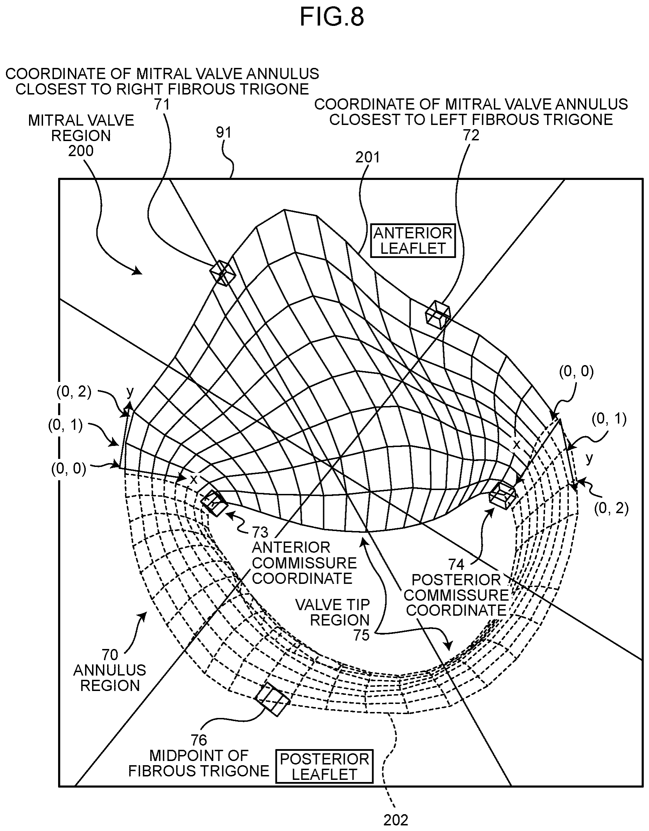

FIG. 8 is a diagram illustrating an example of the definition of a mesh indicating the mitral valve region 200 according to the embodiment. The mitral valve mesh representing the mitral valve region 200 in a mesh image 91 illustrated in FIG. 8 includes an anterior leaflet mesh representing the anterior leaflet region 201 and a posterior leaflet mesh representing the posterior leaflet region 202. For example, the number of grid points of the anterior leaflet mesh is 171 (19 columns × 9 rows), and the number of grid points of the posterior leaflet mesh is 225 (25 columns × 9 rows). Moreover, the mitral valve mesh includes, as components, an annulus region 70, anterior commissure coordinates 73, posterior commissure coordinates 74, respective coordinates of two feature points on the annulus region (for example, coordinates 71 of the mitral valve annulus closest to the right fibrous trigone and coordinates 72 of the mitral valve annulus closest to the left fibrous trigone), a valve tip region 75, and a midpoint 76 of the fibrous trigone. In the example illustrated in FIG. 8, the annulus region 70 corresponds to the grid of row Y=0 in the mitral valve mesh. Moreover, the valve tip region 75 corresponds to the grid of row Y=8 in the mitral valve mesh. In the example illustrated in FIG. 8, the anterior commissure coordinates 73 are coordinates indicating the position of the anterior commissure and correspond to the position of X=0 and Y=8. The posterior commissure coordinates 74 are coordinates indicating the position of the posterior commissure and correspond to the position of X=18 and Y=8 in the example illustrated in FIG. 8. With such definitions, the shape of the mitral valve region 200 is represented by a mesh. The number and the arrangement of grid points of the mesh and the feature points to be extracted illustrated in FIG. 8 are merely examples. The definition of the mesh is not limited thereto.

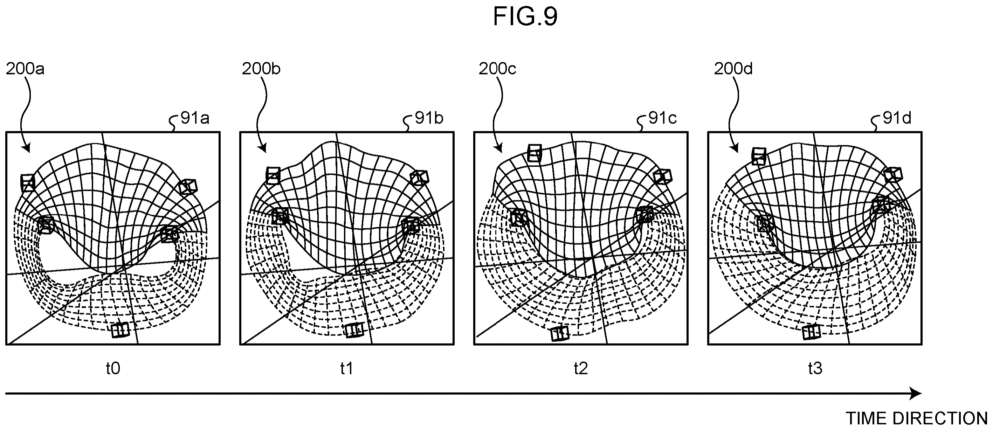

FIG. 9 is a diagram illustrating an example of time-series changes in the shape of the mitral valve mesh representing the mitral valve region 200 according to the embodiment. Mesh images 91a to 91d in FIG. 9 each represent mitral valve regions 200a to 200d specified in the three-dimensional images 90a to 90d illustrated in FIG. 5. As described above, the mitral valve 20 performs regular and periodic reciprocating motion. Hence, the shape of the mitral valve 20 captured in each of the three-dimensional images 90a to 90d differs. The target organ region specification function 152 specifies the shape of the mitral valve regions 200a to 200d at times t0 to t3, by specifying the mitral valve regions 200a to 200d in each of the three-dimensional images 90a to 90d.

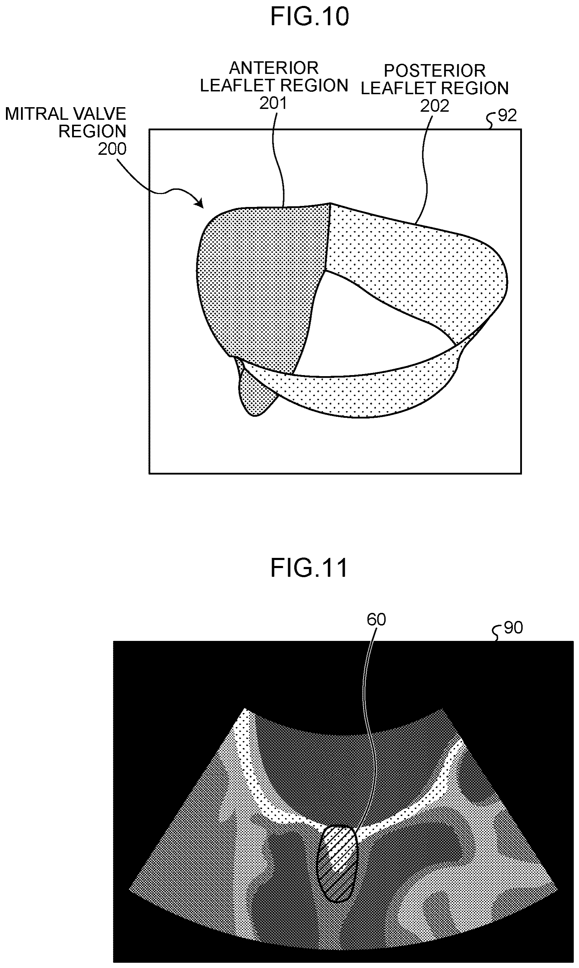

FIG. 10 is a diagram illustrating an example of a method for specifying the mitral valve region 200 as a region according to the embodiment. As a segmentation image 92 illustrated in FIG. 10, the mitral valve region 200 may be specified as a continuous three-dimensional region. With this method also, it is possible to specify the anterior leaflet region 201 and the posterior leaflet region 202 included in the mitral valve region 200.

As the method for specifying the mitral valve region 200 in the three-dimensional image 90, known techniques can be employed. For example, the target organ region specification function 152 may specify the mitral valve region 200, on the basis of the position of the mitral valve 20 manually specified by a user, that is received by the reception function 159 via the input interface 130. Alternatively, the target organ region specification function 152 may specify the mitral valve region 200 on the basis of the anatomical structure extracted by known region extraction techniques. The known region extraction methods include, for example, Otsu's binarization method based on the luminance values of the three-dimensional images 90a to 90d, a region expansion method, a snake method, a graph cut method, a mean shift method, and the like. Moreover, the target organ region specification function 152 may specify the mitral valve region 200 by using a shape model of the mitral valve region 200 that is constructed on the basis of training data prepared in advance by machine learning techniques (including deep learning). The display control function 158 may present to the user the methods described above, and the user may select one of the presented methods. In this case, the target organ region specification function 152 specifies the mitral valve region 200 by the method selected by the user.

Returning to FIG. 4, the device region specification function 153 specifies the region of the device 30 (device region) in each of the three-dimensional images included in the acquired four-dimensional image (step S3). Specifically, in each of the three-dimensional images 90a to 90d included in the 4D TEE image, the device region specification function 153 acquires coordinate information about each pixel where the device 30 is depicted. In other words, the coordinate information about each pixel of the device 30 is coordinate information about each pixel included in the device region.

The device region specification function 153 may specify the coordinate information about each pixel included in the device region as a domain, or may specify it by respective three-dimensional coordinates of two or more points.

FIG. 11 is a diagram illustrating an example of a device region 60 specified as a domain according to the embodiment. The device region specification function 153 may specify, in the three-dimensional image 90, the three-dimensional coordinates in a range corresponding to the contour of the entire device 30 as a domain. Moreover, when the device region specification function 153 specifies the device region 60 as a domain, the device region specification function 153 may specify the device region 60 as a mesh or as a region (segment).

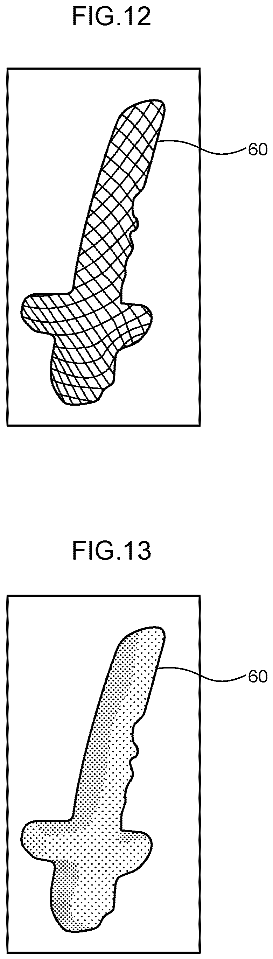

FIG. 12 is a diagram illustrating an example of a method for specifying the device region 60 as a mesh out of the methods for specifying the device region 60 as a domain according to the embodiment. The device region specification function 153 may specify a device region mesh representing the device region 60 by the same method of specifying the mitral valve mesh representing the mitral valve region 200 described with FIG. 7 and FIG. 8. The number and arrangement of the grids of the mesh illustrated in FIG. 12 is merely an example, and it is not limited thereto.

FIG. 13 is a diagram illustrating an example of a method for specifying the device region 60 as a region out of the methods for specifying the device region 60 as a domain according to the embodiment. Similar to the specification of the mitral valve region 200 described in FIG. 10, the device region specification function 153 may specify the device region 60 as a continuous three-dimensional region.

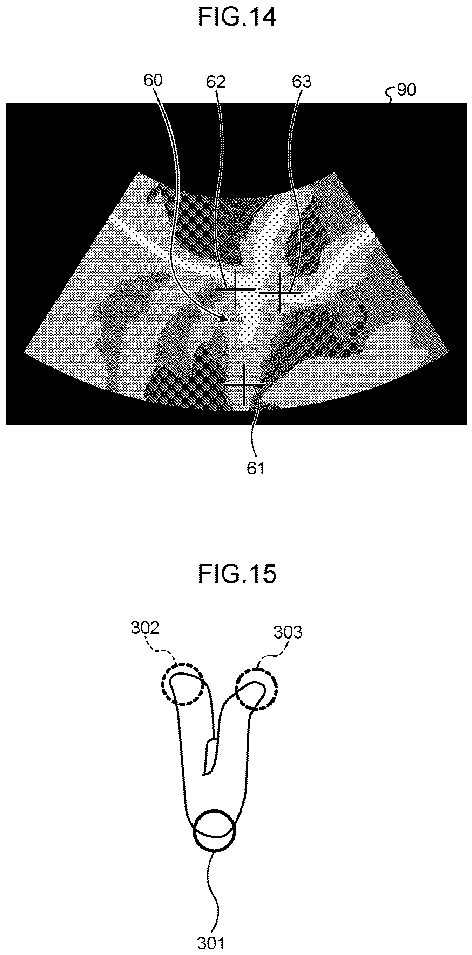

FIG. 14 is a diagram illustrating an example of a method for specifying the device region 60 by respective three-dimensional coordinates 61 to 63 of three points according to the embodiment. In the example illustrated in FIG. 14, the device region 60 is specified by the respective three-dimensional coordinates 61 to 63 of three points in the three-dimensional image 90. However, the number of points indicated by the three-dimensional coordinates is not limited to the example illustrated in FIG. 14 (i.e., three points), and may be any number as long as the three-dimensional coordinates can represent the spatial shape of the entire device 30.

The respective three-dimensional coordinates 61 to 63 of the three points illustrated in FIG. 14 correspond to the three feature points of the device 30.

FIG. 15 is a diagram illustrating an example of feature points 301 to 303 of the device 30 according to the embodiment. As illustrated in FIG. 15, the feature points 301 to 303 are three edge points of the device 30. In general, the device 30 includes bifurcated parts for gripping the mitral valve 20, and a tip end part that connects the bifurcated parts. The two edge points of the bifurcated parts for gripping the mitral valve 20 correspond to the feature points 302 and 303. The length of a line segment that connects between the feature point 302 and the feature point 303 refers to the maximum length of the device 30 in the short-length direction. The edge point at the tip end part of the device 30 corresponds to the feature point 301. By specifying such three edge points as the feature points 301 to 303, the device region specification function 153 can specify the device region 60 corresponding to the entire device 30 in the three-dimensional image 90.

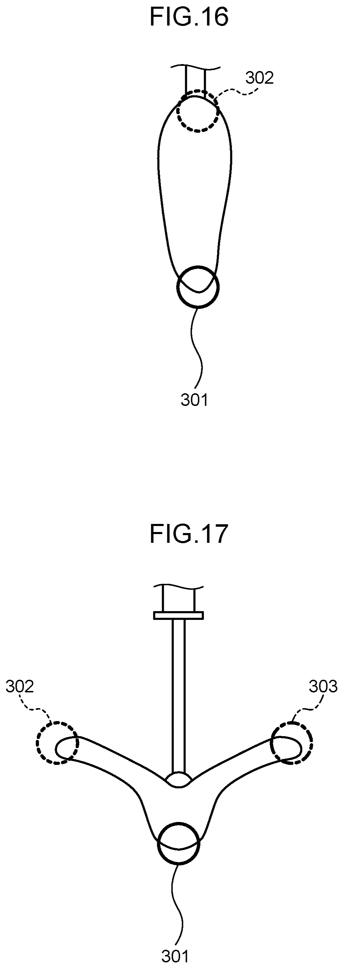

The positions of the edge points of the device 30 may vary with the open/close angle of the device 30 in the three-dimensional image 90. FIG. 16 is a diagram illustrating an example of the feature points 301 and 302 when the device 30 is closed more firmly than that in FIG. 15. As illustrated in FIG. 16, when the device 30 firmly grips the mitral valve 20, the bifurcated parts of the device 30 almost overlap with each other. Hence, in such a situation, the feature points 302 and 303 may be depicted as a single feature point 302. FIG. 17 is a diagram illustrating an example of the feature points 301 to 303 when the device 30 is opened more widely than that in FIG. 15. Even when the open angle of the device 30 is wide as in this case, by specifying the three edge points of the device 30 as the feature points 301 to 303, it is possible to specify the device region 60 corresponding to the entire device 30 in the three-dimensional image 90.

As a method for specifying the device region 60 in the three-dimensional image 90, known techniques can be employed. For example, the device region specification function 153 may specify the device region 60, on the basis of a range manually specified by the user where the device 30 is depicted, or the positions of the feature points 301 to 303 of the device 30, that is received by the reception function 159 via the input interface 130. Alternatively, the device region specification function 153 may specify the device region 60 on the basis of the anatomical structure extracted by using known region extraction techniques. As described above, the known region extraction methods include Otsu's binarization method based on the luminance values of the three-dimensional images 90a to 90d, a region expansion method, a snake method, a graph cut method, a mean shift method, and the like. Moreover, the device region specification function 153 may specify the device region 60 by using a shape model of the device region 60 that is constructed on the basis of training data prepared in advance by machine learning techniques (including deep learning). Alternatively, the device region specification function 153 may specify the device region 60 on the basis of the feature points 301 to 303 of the device 30 extracted by using a known feature point extraction technique. Moreover, the display control function 158 may present the methods described above to the user, and the user may select one of the presented methods. In this case, the device region specification function 153 specifies the device region 60 by using the method selected by the user.

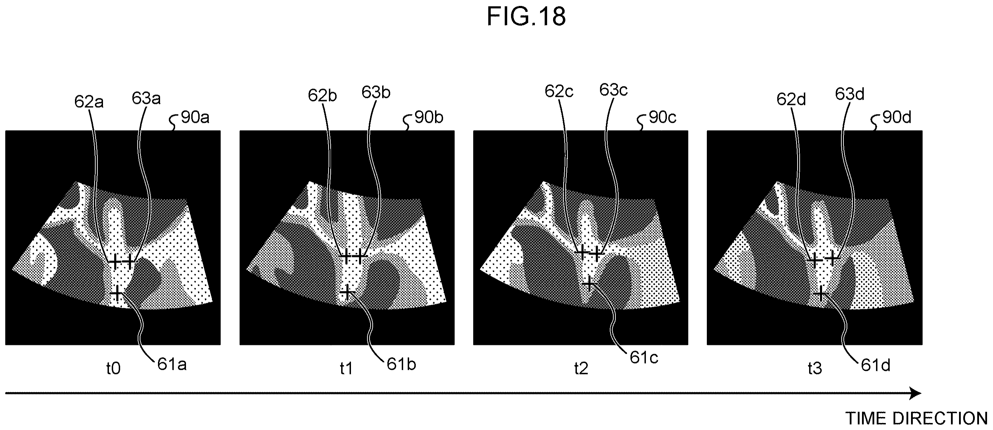

FIG. 18 is a diagram illustrating an example of time-series changes in the positions of the three-dimensional coordinates 61 to 63 that define the device region 60 according to the embodiment. In FIG. 18, the device region specification function 153 specifies, for the device region 60, points with three-dimensional coordinates 61a to 61d, points with three-dimensional coordinates 62a to 62d, and points with three-dimensional coordinates 63a to 63d, as in FIG. 14, in the three-dimensional images 90a to 90d.

The device 30 is attached to the mitral valve 20. Therefore, the three-dimensional coordinates 61 to 63 move toward different positions with the movement of the mitral valve 20 at times t0 to t3. The device region specification function 153 specifies changes in the position of the device 30 at times t0 to t3, by specifying respective points with the three-dimensional coordinates 61a to 61d, 62a to 62d, and 63a to 63d in the three-dimensional images 90a to 90d.

Returning to FIG. 4, the attention coordinate specification function 154 specifies the attention coordinates of the device 30 in the device region 60 that has been specified by the device region specification function 153 in each of the three-dimensional images 90a to 90d (step S4).

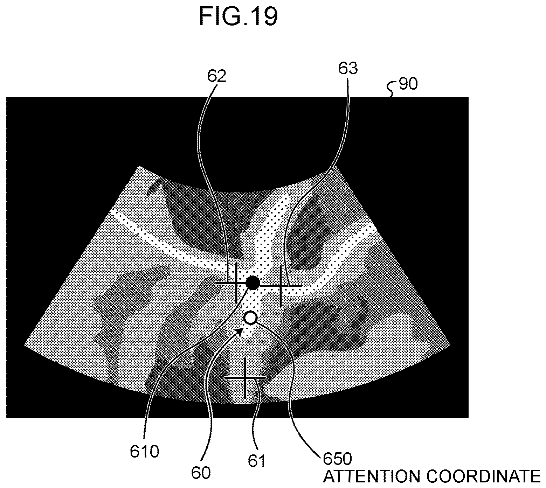

FIG. 19 is a diagram illustrating an example of attention coordinates 650 according to the embodiment. The attention coordinates 650 represent one of points each indicated by the three-dimensional coordinates included in the device region 60. In the example illustrated in FIG. 19, the attention coordinates 650 correspond to the center of gravity of the three-dimensional coordinates 61 to 63 of three points corresponding to the edges of the device 30. Specifically, the attention coordinate specification function 154 specifies a midpoint 610 between two points of the three-dimensional coordinates 62 and 63 corresponding to the bifurcated parts of the device 30. Then, the attention coordinate specification function 154 sets, as the attention coordinates 650, coordinates of the midpoint between the midpoint 610 and a point of the three-dimensional coordinates 61 corresponding to the tip end of the device 30. The attention coordinates 650 illustrated in FIG. 19 is merely an example, and it is not limited thereto. As another example, the attention coordinate specification function 154 may set, as the attention coordinates 650, the three-dimensional coordinates 61 corresponding to the tip end of the device 30.

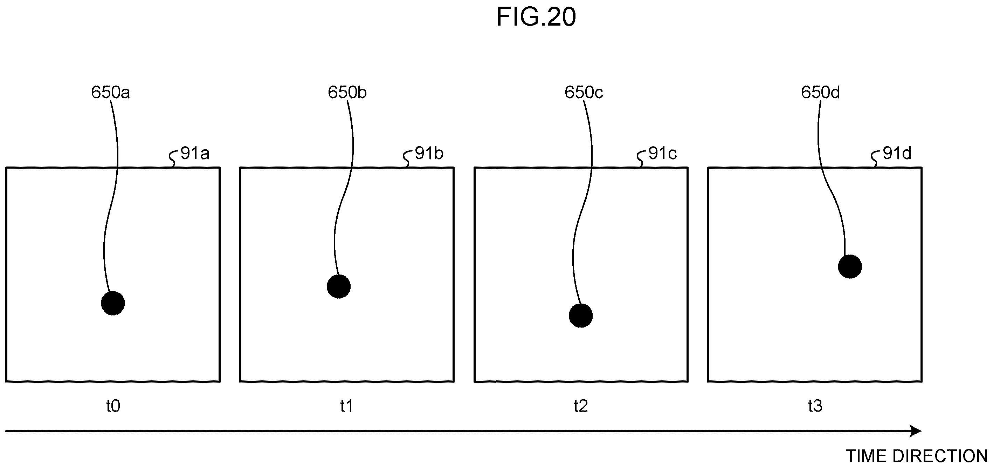

FIG. 20 is a diagram illustrating an example of time-series changes in the positions of attention coordinates 650a to 650d according to the embodiment. In FIG. 20, illustration of the three-dimensional image 90 is omitted. However, similar to the three-dimensional coordinates 61a to 61d, 62a to 62d, and 63a to 63d in FIG. 18, the positions of the attention coordinates 650a to 650d are specified in the three-dimensional images 90a to 90d.

The device 30 is attached to the mitral valve 20, so that the attention coordinates 650a to 650d move to different positions with the movement of the mitral valve 20 at times t0 to t3. In other words, the attention coordinates 650a to 650d represent multiple pixels included in the device region 60 and indicate the changes in the position of the device 30 at times t0 to t3.

Returning to FIG. 4, the attention direction specification function 155 specifies the attention direction of the device 30 on the basis of the device region 60 specified by the device region specification function 153 in each of the three-dimensional images 90a to 90d (step S5).

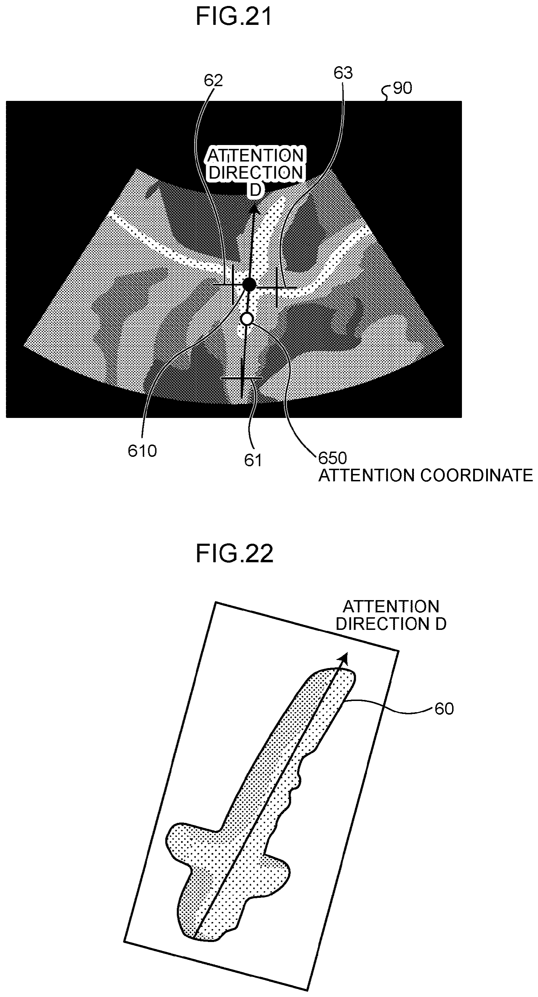

FIG. 21 is a diagram illustrating an example of an attention direction D according to the embodiment. In the example of FIG. 21, the attention direction D is the long axis direction of the device 30. In general, the long axis direction of the device 30 is a direction following the blood flow through the mitral valve 20. The attention direction specification function 155 may specify the long axis direction of the device 30, on the basis of the geometric relation between the three-dimensional coordinates of multiple points that define the device region 60.

In the example illustrated in FIG. 21, the attention direction specification function 155 specifies, as the attention direction D, the extending direction of a line segment from the attention coordinates 650 toward the midpoint 610. The line segment connects a point of the attention coordinates 650 and the midpoint 610 between respective points of the three-dimensional coordinates 62 and 63 corresponding to the two feature points 302 and 303 of the bifurcated parts of the device 30.

For the method of specifying the attention direction D, various known techniques can be employed. For example, the attention direction specification function 155 may specify the attention direction D on the basis of the user operation received by the reception function 159 via the input interface 130.

Alternatively, the attention direction specification function 155 may specify a first principal component obtained through the principal component analysis with respect to the coordinate information about each pixel included in the device region 60 of each of the three-dimensional images 90a to 90d, as the attention direction D. FIG. 22 is a diagram illustrating an example of a specification method of the attention direction D according to the embodiment in a case where the device region 60 is specified as a domain. In the example illustrated in FIG. 22, the attention direction specification function 155 specifies the attention direction D through the principal component analysis with respect to the coordinate information about each pixel included in the device region 60.

Alternatively, the attention direction specification function 155 may specify the attention direction D by using a model for estimating the long axis direction of the device 30 that is constructed on the basis of training data prepared in advance by machine learning techniques (including deep learning). The method for specifying the attention direction is not limited thereto. Any method that specifies the typical moving direction of the reciprocating motion of an organ that performs reciprocating motion in the three-dimensional space can be employed. The display control function 158 may present plural methods to the user, and the user may select one of the presented methods. In this case, the attention direction specification function 155 specifies the attention direction D by the method selected by the user.

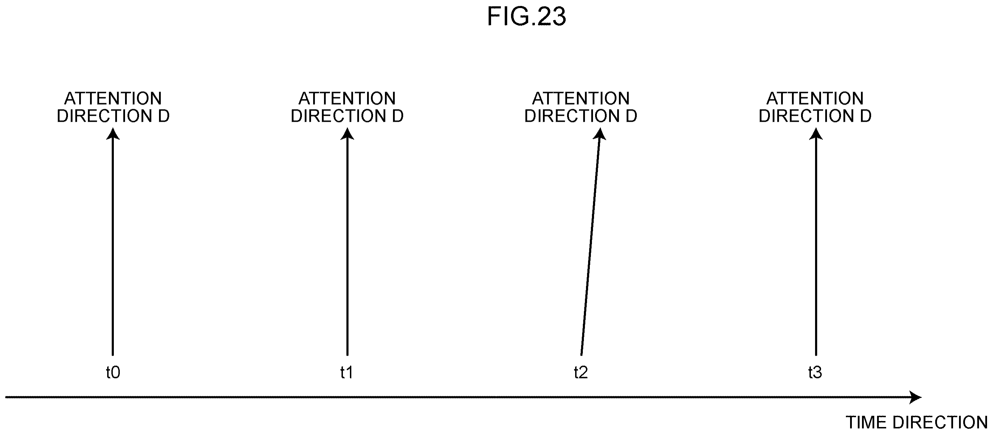

FIG. 23 is a diagram illustrating an example of time-series changes in the attention direction D according to the embodiment.

Because the mitral valve 20 and the device 30 attached to the mitral valve 20 operate three-dimensionally in the three-dimensional space, the orientation of the attention direction D also differs at times t0 to t3. In FIG. 23, the attention direction D is represented in a two-dimensional direction. However, in practice, the attention direction D changes three-dimensionally in each time phase.

Returning to FIG. 4, next, the average coordinate specification function 156 specifies the average coordinates of the mitral valve region 200 at times t0 to t3, when the three-dimensional images 90a to 90d included in the four-dimensional image are captured (step S6).

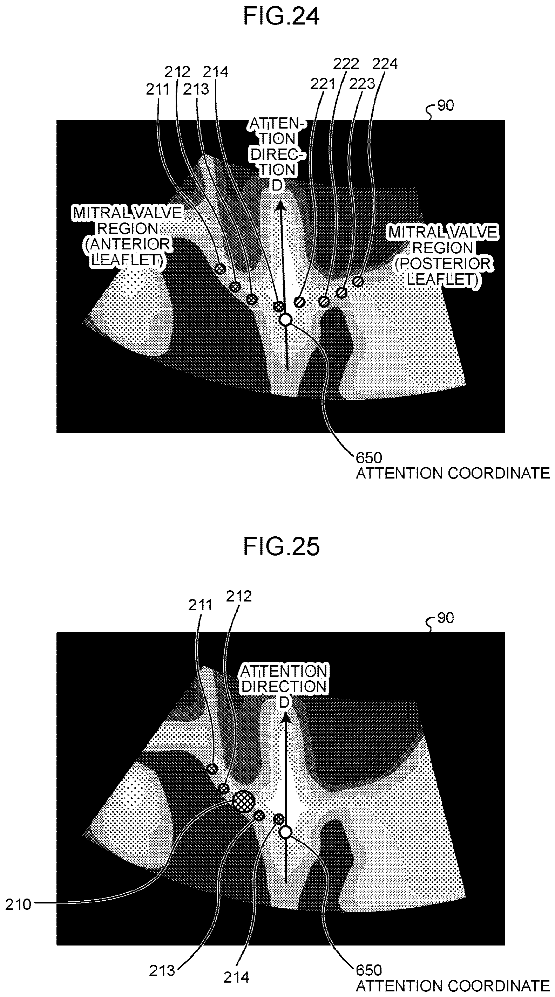

FIG. 24 is a diagram schematically illustrating an example of coordinates 211 to 214 and 221 to 224 of pixels included in the mitral valve region 200 in the three-dimensional image 90 according to the embodiment. The average coordinate specification function 156 may specify the coordinates 211 to 214 and 221 to 224 of the pixels included in the mitral valve region 200, and specify the average of the specified coordinates 211 to 214 and 221 to 224, as the average coordinates.

The average coordinate specification function 156 may specify the average coordinates in either one of the anterior leaflet 21 or the posterior leaflet 22 of the mitral valve 20, instead of in the entire mitral valve 20. FIG. 25 is a diagram illustrating an example of average coordinates 210 of the coordinates 211 to 214 of pixels included in the anterior leaflet region of the mitral valve region 200 in the three-dimensional image 90 according to the embodiment.

Returning to FIG. 4, the variation calculation function 157 specifies an amount of variation of the distance in the attention direction between the average coordinates 210 and the attention coordinates 650 (step S7).

In one example the variation calculation function 157 specifies the maximum position in the attention direction D and the minimum position in the attention direction D from among the respective average coordinates 210 at times t0 to t3.

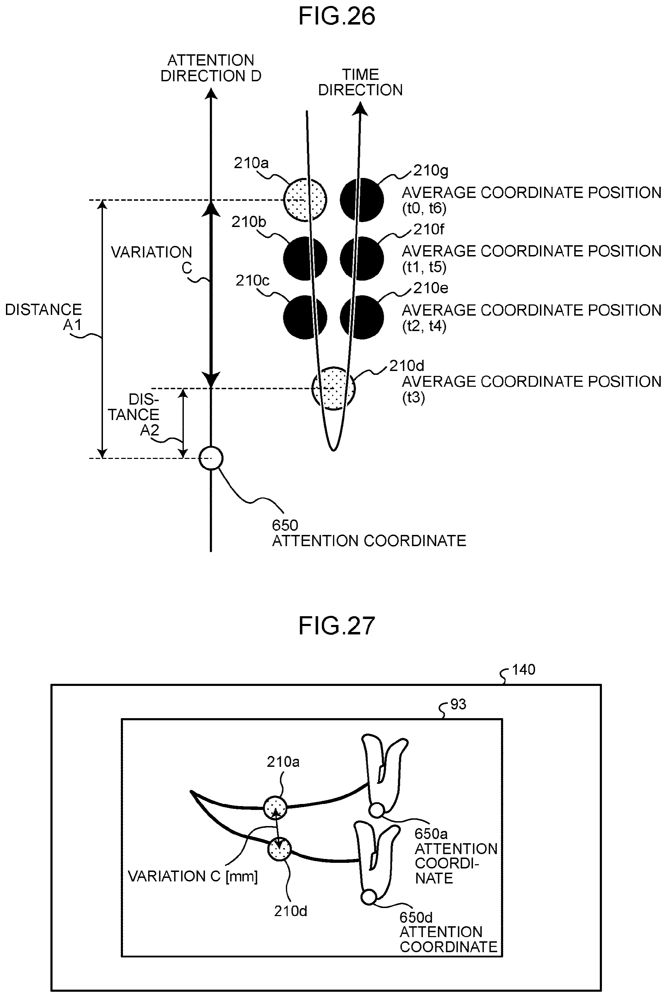

FIG. 26 is a diagram illustrating an example of positions of average coordinates 210a to 210d at times t0 to t6 with respect to the attention direction D according to the embodiment. The times t0 to t3 have been described as examples, whereas, in FIG. 26, times t4 to t6 are added for describing the longer period of time. Times t4 to t6 are times subsequent to time t3 and times when different three-dimensional images 90 are captured.

As described above, the mitral valve 20 performs periodic reciprocating motion. Thus, the average coordinates 210 of the mitral valve region 200 reciprocate in the attention direction D with the lapse of time. In the example illustrated in FIG. 26, the average coordinates 210 move from the position at time t0 (average coordinates 210a) in the direction opposite to the attention direction D at times t1 to t3 (average coordinates 210b to 210d). Then, the reciprocating motion reaches halfway point at time t3. Thereafter, the average coordinates 210 moves in the attention direction D from the position at time t3 (average coordinates 210d) to times t4 to t6 (average coordinates 210e to 210g). At time t6, the average coordinates 210g returns to the position at time t0 (average coordinates 210a).

In the example illustrated in FIG. 26, the average coordinates 210a and 210g at times t0 and t6 are the coordinates located on the most extended direction of the attention direction D. Therefore, the variation calculation function 157 sets the average coordinates 210a and 210g as the maximum position in the attention direction D. In the example illustrated in FIG. 26, the average coordinates 210d at time t3 are the coordinates located on the side opposite to the most extended direction of the attention direction D. The variation calculation function 157 sets the average coordinates 210d as the minimum position in the attention direction D.

In FIG. 26, the attention coordinates 650 are illustrated as located in a single position, whereas, in practice, the attention coordinates 650 also move with the movement of the device 30 attached to the mitral valve 20. However, due to the expansion and contraction of the mitral valve 20, difference arises between the moving amount of the average coordinates 210 and the moving amount of the device 30. Therefore, the distance between the attention coordinates 650 and the average coordinates 210 in the attention direction D changes between time phases. Moreover, the maximum position of the average coordinates of the mitral valve 20 is not limited to only coordinates of a single point, and a given range may be set as the "maximum position" of the mitral valve 20. For example, a group of points of coordinates with a prescribed threshold or more in the attention direction D may be set as a range of the "maximum position" of the mitral valve 20. In this case, the coordinates to be used for calculating the amount of variation, which will be described later, may be selected from the average coordinates of the mitral valve 20 included in the above-noted range.

The variation calculation function 157 obtains a distance A1 between a point of the attention coordinates 650 and the maximum position (corresponding to the average coordinates 210a and 210g) in the attention direction D. Moreover, the variation calculation function 157 obtains a distance A2 between a point of the attention coordinates 650 and the minimum position (corresponding to the average coordinates 210d) in the attention direction D. The variation calculation function 157 obtains, as an amount of variation C, the absolute value of a difference between the distance A1 and the distance A2. The distance A1 is an example of a first distance in the present embodiment. The distance A2 is an example of a second distance in the present embodiment. Since the average coordinates 210 represent the mitral valve region 200, the distances A1 and A2 each represent the distance between the attention coordinates 650 and the mitral valve region 200 at times t0 and t3.

Returning to FIG. 4, next, the display control function 158 causes the display 140 to display the amount of variation C calculated by the variation calculation function 157 (step S8). The amount of variation C is an example of an index relating to the state of the mitral valve 20.

FIG. 27 is a diagram illustrating an example of a display mode of the amount of variation C according to the embodiment. As illustrated in FIG. 27, the display control function 158 may cause the display 140 to display the average coordinates 210a and 210d at times t0 and t3, and the attention coordinates 650a and 650d at times t0 and t3, along with the amount of variation C from times t0 to time t3. In this case, the display control function 158 may cause the display 140 to superimpose the value of the amount of variation C on an image 93 where the mitral valve 20 and the device 30 at times t0 and t3 are depicted. The display mode of the amount of variation C is not limited to the example illustrated in FIG. 27. The display control function 158 may cause the display 140 to display a graph indicating time-series changes in the amount of variation C of the distance between the attention coordinates 650 and the average coordinates 210. The processing of the flowchart ends here. The processing of the flowchart is repeatedly executed during the procedure until the physician releases the shaft 31. Hence, the physician can continuously check the index relating to the state of the mitral valve 20 during the procedure.

In this manner, the medical image processing apparatus 100 of the present embodiment specifies the mitral valve region 200 and the device region 60 in each of the images of the mitral valve 20 of a subject captured in time series. Based on the specified mitral valve region 200 and the device region 60, the medical image processing apparatus 100 calculates an index relating to the state of the mitral valve 20 to which the device 30 is attached. The index is calculated from change in the relative position of the device 30 with respect to the mitral valve 20 in the specific attention direction D. Therefore, according to the medical image processing apparatus 100 of the present embodiment, when the device 30 for treatment is to be attached to the mitral valve 20 of a subject, it is possible to calculate an index relating to the state of the mitral valve 20 with high accuracy during the procedure. Therefore, according to the medical image processing apparatus 100 of the present embodiment, it is possible to support the decision making of the physician on releasing the shaft 31, changing the attachment position of the device 30, stopping the procedure, or the like.

In a comparative example, when the tension or the like of the mitral valve is estimated by simulating the time change in the three-dimensional shape of the mitral valve or the blood flow through the mitral valve, the calculation cost is expensive, and it is difficult to use the method during the procedure. In contrast, according to the method of the present embodiment, an index indirectly indicating the tension or the like of the mitral valve 20 can be calculated as a numerical value, from the geometric positional relation between the mitral valve 20 and the device 30. Hence, the calculation cost can be reduced, and it is possible to use the method during the procedure.

Moreover, the medical image processing apparatus 100 of the present embodiment specifies the attention coordinates 650 being the three-dimensional coordinates of one point included in the device region 60 and the attention direction D being the long axis direction in the three-dimensional space of the device 30, in each of the three-dimensional images 90a to 90d of the mitral valve 20 captured in time series. The medical image processing apparatus 100 calculates, as an index relating to the state of the mitral valve 20, the amount of variation C of the distance in the attention direction D between the attention coordinates 650 and the mitral valve 20 at different time points (times t0 to t3). The shape of the device 30 does not change over time. Therefore, as in the medical image processing apparatus 100 of the present embodiment, by calculating the amount of variation C based on the attention coordinates 650 on the basis of the geometrical shape of the device 30, it is possible to reduce the effects of changes in the coordinates in the three-dimensional space regarding the movement other than that of the mitral valve 20 such as the body movement of the subject, the movement of the ultrasonic probe caused by breathing, and heartbeat. Moreover, according to the medical image processing apparatus 100 of the present embodiment, by setting the attention direction D on the basis of the geometric shape of the device 30 that does not change over time as the reference direction for calculating the amount of variation C, it is possible to calculate the amount of variation C of the coordinates in the three-dimensional space in the blood flowing direction that is to be noted most with high accuracy.

The medical image processing apparatus 100 of the present embodiment specifies the maximum position and the minimum position with respect to the attention direction D from among respective positions of the average coordinates 210 of the mitral valve 20 at the different time points, in the mitral valve region 200 specified in each of the three-dimensional images 90a to 90d of the mitral valve 20. The medical image processing apparatus 100 of the present embodiment calculates, as the amount of variation C, the absolute value of the difference between the distance A1 and the distance A2. The distance A1 is a distance between the attention coordinates 650 and the maximum position in the attention direction D. The distance A2 is a distance between the attention coordinates 650 and the minimum position in the attention direction D. Therefore, according to the medical image processing apparatus 100 of the present embodiment, it is possible to easily obtain the change in the positional relation between the mitral valve 20 and the device 30 due to the periodic reciprocating motion of the mitral valve 20.

The medical image processing apparatus 100 of the present embodiment specifies the device region 60 by using the three feature points 301 to 303 corresponding to the three edge points of the device 30 and specifies the center of gravity of the three feature points 301 to 303 as the attention coordinates 650. From among the three feature points 301 to 303, the medical image processing apparatus 100 may specify the extending direction of a line segment from the attention coordinates 650 toward the midpoint 610 as the attention direction D, the line segment connecting between the attention coordinates 650 and the midpoint 610 between points of the three-dimensional coordinates 62 and 63 of the feature points 302 and 303 corresponding to the two edge points of the device 30 in the short-length direction. Therefore, according to the medical image processing apparatus 100 of the present embodiment, by utilizing the shape of the device 30 that does not change over time, it is possible to stably specify the attention direction D with high accuracy.

The medical image processing apparatus 100 of the present embodiment may specify, as the attention direction D, the first principal component obtained through the principal component analysis with respect to the coordinate information about each pixel included in the device region 60 specified in each of the three-dimensional images 90a to 90d of the mitral valve 20. According to the medical image processing apparatus 100 of the present embodiment, even when such a method is employed, it is possible to stably specify the attention direction D with high accuracy, by utilizing the shape of the device 30 that does not change over time.

The medical image processing apparatus 100 of the present embodiment displays, on the display 140, the calculated amount of variation C along with an image indicating the maximum position and the minimum position of the attention coordinates 650. Alternatively, the medical image processing apparatus 100 of the present embodiment display, on the display 140, a graph indicating time-series changes in the calculated amount of variation C. Therefore, the medical image processing apparatus 100 of the present embodiment can allow the physician or the like to visually recognize the index relating to the state of the mitral valve 20.

### First Modification

In the embodiment described above, the medical image processing apparatus 100 calculates, as an index relating to the state of the mitral valve 20, the amount of variation C between time phases with respect to the distance in the attention direction D between the average coordinates 210 of the mitral valve region 200 and the attention coordinates 650 of the device 30 in the three-dimensional images 90a to 90d. The method for specifying the amount of variation of the three-dimensional distance between the mitral valve 20 and the device 30 between time phases is not limited thereto.

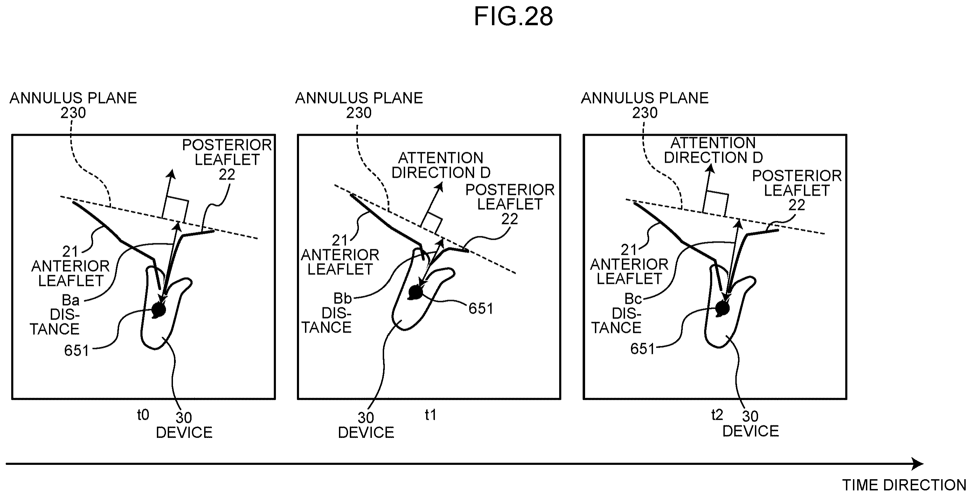

FIG. 28 is a diagram illustrating an example of a specification method of three-dimensional distance between the mitral valve 20 and the device 30 between time phases according to a first modification. In the example illustrated in FIG. 28, the variation calculation function 157 calculates distances Ba, Bb, and Bc between an annulus plane 230 and attention coordinates 651 at times t0, t1, and t2, in each of the three-dimensional images 90a to 90c captured at different times t0 to t2. Then, the variation calculation function 157 calculates the amount of variation of the distances Ba, Bb, and Bc between times t0, t1, and t2, as an index relating to the state of the mitral valve 20.

In the present modification, the target organ region specification function 152 specifies the annulus plane 230 in each of the three-dimensional images 90a to 90c captured at different times t0 to t2. The annulus plane 230 is used as a reference for the position of the mitral valve 20, to specify the three-dimensional distance between the mitral valve 20 and the device 30. The annulus plane 230 is an example of a reference plane in the present modification.

In one example, the target organ region specification function 152 may specify the least-squares plane of coordinates corresponding to the annulus of the mitral valve 20 in the mitral valve region 200 as the annulus plane 230. The specification method of the annulus plane 230 is not limited to the method described above, and may be any method that can specify a plane approximate to the annulus of the mitral valve 20.

The mitral valve 20 performs periodic reciprocating motion in the normal direction of the annulus plane 230. Therefore, by utilizing such a property of the mitral valve 20, the attention coordinate specification function 154 of the present modification specifies the normal direction of the annulus plane 230 as the attention direction D.

In the present modification, the attention coordinate specification function 154 may specify the attention coordinates 651 by the same method as that in the embodiment described above, or may specify the attention coordinates 651 indicating the three-dimensional position of the device 30 by other methods.

In this manner, the medical image processing apparatus 100 of the present modification specifies the annulus plane 230 of the mitral valve 20 as the reference plane, and specifies the amount of variation of the distance Ba to Bc between the annulus plane 230 and the attention coordinates 651 in the attention direction D between different time points, as an index. With such a method, it is also possible to easily obtain the change in the positional relation between the mitral valve 20 and the device 30 due to the periodic reciprocating motion of the mitral valve 20.

Moreover, the medical image processing apparatus 100 of the present modification specifies the normal direction of the annulus plane 230 as the attention direction D, and specifies the amount of variation of the distance Ba to Bc between the annulus plane 230 and the attention coordinates 651 in the attention direction D between different time points, as an index. In this manner, by a method other than the method for obtaining the long-length direction of the device 30, it is possible to specify the direction of the periodic movement of the mitral valve 20 and the device 30.

### Second Modification

In the embodiments described above, the target organ is assumed to be a heart valve, in particular, the mitral valve 20. However, the target organ is not limited thereto. The target organ may be another heart valve, or may be organs other than the heart valve, as long as the organs move regularly. For example, the target organ may also be a diaphragm, an eyelid, joints (arms; hips; adduction, abduction, internal rotation, and external rotation of foot), etc.

### Third Modification

In the embodiments described above, a specific example of the device 30 to be attached to the target organ is MitraClip (registered trademark) or PASCAL (Edwards Lifesciences) that can be attached to the mitral valve 20. However, the device 30 is not limited thereto. Any treatment device attachable to the target organ that performs reciprocating motion in the three-dimensional space and that restrains the reciprocating motion of the target organ may be used as the device 30.

### Fourth Modification

In the embodiments described above, the calculation processing of an index relating to the state of the mitral valve 20 is executed when the procedure of attaching the device 30 to the mitral valve 20 is being performed. However, the timing of the calculation is not limited thereto. For example, the calculation processing of an index relating to the state of the mitral valve 20 may be performed for the purpose of periodical inspection on the state of the mitral valve 20 after the device 30 is attached.

### Fifth Modification

In the embodiments described above, the number of the device 30 to be attached to the mitral valve 20 is one. However, the number of the device 30 to be attached to the mitral valve 20 may be two or more. The tension on the mitral valve 20 may change with the number of the device 30 to be attached to the mitral valve 20.

The size of each device 30 may vary with the product. The tension on the mitral valve 20 increases with increase in the width and height of the device 30. Therefore, even if the amount of variation C of each device 30 is the same, the state of the mitral valve 20 may be different.

Therefore, the variation calculation function 157 may correct the calculated index (for example, the amount of variation C) in accordance with the respective sizes or the number of the devices 30. As a method of such correction, a prescribed correction value may be multiplied by the amount of variation C in accordance with the respective sizes or the number of the devices 30.

### Sixth Modification

In the embodiments described above, the variation calculation function 157 calculates the numerical value of the amount of variation C as an index relating to the state of the target organ. However, the method for specifying an index is not limited thereto. For example, an index may be specified by specifying the level indicating the magnitude of the amount of variation C in a stepwise manner. Moreover, for example, the variation calculation function 157 may represent the magnitude of the amount of variation C based on the prescribed criteria such as large, medium, and small.

The various types of data handled in the present disclosure are typically digital data.

According to at least one of the embodiments described above, when a device for treatment is to be attached to the target organ of a subject, it is possible to calculate an index relating to the state of the target organ with high accuracy during the procedure.

While certain embodiments have been described, these embodiments have been presented by way of example only, and are not intended to limit the scope of the inventions. Indeed, the novel embodiments described herein may be embodied in a variety of other forms; moreover, various omissions, substitutions and changes in the form of the embodiments described herein may be made without departing from the scope of the inventions as defined by the appended claims.

## Claims

1. A medical information processing apparatus (100) comprising processing circuitry (150) configured to:
acquire a plurality of images of a target organ (20) captured in time series;
specify, in each of the images, a target organ region (200) where the target organ (20) is depicted;
specify, in each of the images, a device region (60) where a device (30) attached to the target organ (20) is depicted; and
specify, based on the target organ region (200) and the device region (60), an index relating to a state of the target organ (20) to which the device (30) is attached, the index being specified from change in a relative position of the device (30) with respect to the target organ (20) in a specific attention direction (D).

2. The medical information processing apparatus (100) according to claim 1, wherein
the images are three-dimensional images of the target organ (20) captured in time series,
the processing circuity (150) is further configured to
specify, in each of the images, attention coordinates (650) being three-dimensional coordinates of one point included in the device region (60), and
specify, in each of the images, a long axis direction of the device (30) in a three-dimensional space as the attention direction based on the device region (60), and
the index is an amount of variation (C) of a distance between the attention coordinates (650) and the target organ region (200) in the attention direction (D).

3. The medical information processing apparatus (100) according to claim 2, wherein
the processing circuitry (150) is further configured to
acquire, at different time points, the images of the target organ (20) captured in time series,
specify average coordinates (210) of the target organ (20) at each of the different time points, the average coordinates (210) being specified from the target organ region (200) specified in each of the images,
specify a maximum position and a minimum position in the attention direction (D) from among the average coordinates (210) at each of the different time points, and
calculate, as the amount of variation (C), an absolute value of a difference between a first distance (A1) and a second distance (A2), the first distance (A1) being a distance between the attention coordinates (650) and the maximum position in the attention direction (D), the second distance (A2) being a distance between the attention coordinates (650) and the minimum position in the attention direction (D).

4. The medical information processing apparatus (100) according to claim 2, wherein the processing circuitry (150) is configured to specify, as the attention direction (D), a first principal component obtained through a principal component analysis with respect to coordinate information about each pixel included in the device region (60) specified in each of the images.

5. The medical information processing apparatus (100) according to claim 2, wherein the processing circuitry (150) is further configured to
specify the device region (60) by three feature points (301, 302, 303) corresponding to three edge points of the device (30),
specify, as the attention coordinates (650), a center of gravity of the three feature points (301, 302, 303), and
specify, as the attention direction (D), an extending direction of a line segment from the attention coordinates (650) toward a midpoint (610), the line segment connecting between the attention coordinates (650) and the midpoint (610) that connects respective three-dimensional coordinates (62, 63) of two feature points (302, 303) corresponding to two edge points in a short-length direction of the device (30) among the three feature points (301, 302, 303).

6. The medical information processing apparatus (100) according to claim 3, wherein the processing circuitry (150) is further configured to
display, on a display (140), the index along with an image indicating the maximum position and the minimum position of the attention coordinates (650), or
display, on the display (140), a graph indicating a time-series change in the calculated index.

7. The medical information processing apparatus (100) according to claim 1, wherein the processing circuitry (150) is further configured to correct the index in accordance with the size of the device (30) or the number of the devices (30).

8. The medical information processing apparatus (100) according to claim 1, wherein
the processing circuitry (150) is further configured to
specify a reference plane (230) of the target organ (20), specify, in each of the images, attention coordinates (650) being three-dimensional coordinates of one point included in the device region (60), and
specify a normal direction of the reference plane (230) as the attention direction (D), and
the index relating to the state of the target organ (20) is an amount of variation (C) of a distance between the reference plane (230) and the attention coordinates (650) in the attention direction.

9. The medical information processing apparatus (100) according to claim 1, wherein
the target organ (20) is a mitral valve (20) of the heart,
the device (30) serves to grip tip ends of an anterior leaflet and a posterior leaflet of the mitral valve (20), and
the index is a numerical value indirectly indicating tension of the mitral valve (20) in a state of being gripped by the device (30).

10. A medical information processing method implemented by a computer (100), the method comprising:
acquiring (S1) a plurality of images of a target organ captured in time series;
specifying (S2), in each of the images, a target organ region where the target organ is depicted;
specifying (S3), in each of the images, a device region where a device attached to the target organ is depicted; and
specifying (S7), based on the target organ region and the device region, an index relating to a state of the target organ to which the device is attached, the index being specified from change in a relative position of the device with respect to the target organ in a specific attention direction.
